# EUROPEAN PATENT APPLICATION

(11) **EP 2 798 968 A1**
(43) Date of publication of application: **05.11.2014**
(21) Application number: 13741176.5
(22) Date of filing: 23.01.2013
(51) Int. Cl.: A24F 47/00, A24F 1/28, A61M 15/06

(54) **NON-COMBUSTION FLAVOR INHALATION APPARATUS**

(30) Priority: 24.01.2012 JP 2012012003
(71) Applicant: Japan Tobacco Inc., Tokyo 105-8422 (JP)
(72) Inventor: SUZUKI, Akihiko, Tokyo 130-8603 (JP); UCHII, Kimitaka, Tokyo 130-8603 (JP); YAMADA, Manabu, Tokyo 130-8603 (JP)
(74) Representative: Isarpatent
(86) International application number: PCT/JP2013/051357
(87) International publication number: WO 2013/111792

(57) **Abstract**

A non-combustion flavor inhalation apparatus has a flavor generating body for imparting flavor to an air current that enters from one end and exits from another end, an inhalation unit for a user to use for inhalation and disposed on the other end, a heat source for generating heat by means of an oxidation reaction and for raising the temperature of the flavor generating body, and a casing for holding the flavor generating body, the inhalation unit and the heat source, wherein the casing contains an air adjustment mechanism capable of reversibly changing the contact area of the heat source and the air used for the oxidation reaction.

## Description

### [TECHNICAL FIELD]

The present invention relates to a non-burning type flavor inhaler having a heat source generating chemical reaction heat, especially oxidation reaction heat, where a user inhales a flavor vaporized from a flavor generating body.

### [BACKGROUND ART]

For example, there is a smoking article disclosed in Patent Literature 1 as this kind of the flavor inhaler utilizing the chemical reaction heat. The smoking article has a heat chamber using metallic oxidation heat as a heat source, and the heat chamber disposed on a coaxial direction with a tobacco leaf. The smoking article makes the user to enjoy the flavor without burning, by heating the tobacco leaf using the heat source up to a suitable temperature where the tobacco leaf does not burn.

As a technique for using the oxidation reaction heat, known are techniques related to a so-called disposable body warmer. For example, Patent Literature 2 discloses the disposable body warmer achieves a desirable heat generation temperature by controlling compositions of iron powder, activated carbon, water, and salt which forms a heat generation body. Patent Literature 3 discloses the disposable body warmer varies heat generation temperature by adhering a plurality of film covers having different size of holes onto a surface of a bag housing the heat generation body in a laminated manner that the film covers are laminated in order of decreasing ventilation characteristic.

However, in the smoking article disclosed in Patent Literature 1, once the oxidation reaction starts and then the reaction autonomously proceeds until the oxidation reaction ends. Therefore, the needs cannot be achieved that the user wants to enjoy the flavor after a while by suspending the inhale in the middle. That is, the smoking article disclosed in Patent Literature 1 is a smoking article of one time use type, and it is wasteful since multiple times of use are not cared.

Further, the technique disclosed in Patent Literature 1 has difficulty to control a time-shift of a heated temperature by the heat source (hereinafter it may be called a temperature profile). Therefore, it is obvious from Fig. 5 in Patent Literature 1, for example, a drop in temperature is remarkable after the heat source reaches the maximum temperature, and therefore it is difficult to stably provide a flavor component to the user.

On the other hand, Patent Literatures 2 and 3 disclose a technique for a temperature control by the heat generation body using oxidation reaction. However, Patent Literatures 2 and 3 relate to an improvement of the disposable body warmer applied for a skin of human in the first place, and a preferable temperature profile for the flavor inhaler is completely different from the disposable body warmer. That is, in case of the flavor inhaler, the temperature profile is desirable that the temperature is kept in a temperature range suitable for providing the flavor component after raised rapidly up to a suitable temperature in the beginning of use. On the contrary, since Patent Literatures 2 and 3 disclose the technique completely different from such technical viewpoint, these documents should not be referred.

Accordingly, an object of the present invention is to provide a non-burning type flavor inhaler which can control a heat source in a suitable temperature range in use and still further can be used for multiple times.

### [CITATION LIST]

### [PATENT LITERATURE]

[Patent Literature 1]: Japanese Patent Application Publication No. 2011-509667
[Patent Literature 2]: Japanese Patent Publication No. 4405437
[Patent Literature 3]: Japanese Patent Application Publication No. Heisei 10-108875

### [SUMMARY OF INVENTION]

A non-burning type flavor inhaler according to a first feature comprises: a flavor generating body imparting a flavor to an air flow entering form an one end side and exiting from an opposite end side, a mouthpiece for inhaling by a user arranged at the opposite end side, a heat source producing heat by oxidation reaction and raising a temperature of the flavor generating body, and a casing member holding the flavor generating body, the mouthpiece, and the heat source, wherein the casing member includes an air adjusting structure which reversibly changes an area of contact between the heat source and an air used for the oxidation reaction.

In the first feature, the air adjusting structure includes; a first cylindrical member housing the heat source and the flavor generating body, the first cylindrical member having a first opening, and a second cylindrical member provided at an outside of the first cylindrical member, the second cylindrical member having a second opening, and the first cylindrical member and the second cylindrical member have a configuration being relatively displaceable so as to change an overlapping area of the first opening and the second opening among at least two kinds of area sizes.

In the first feature, the heat source is arranged to cover an outer peripheral of the flavor generating body, and the casing member is arranged to cover the heat source.

In the first feature, the air adjusting structure includes; a first cylindrical member provided to cover the heat source, the first cylindrical member having a first opening for contacting the air to the heat source, a second cylindrical member having a second opening having an opening area equivalent to the first opening and a third opening having an opening area smaller than the first opening, and the first cylindrical member and the second cylindrical member have a configuration being relatively displaceable to a position where the second opening overlaps the first opening, a position where the third opening overlaps the first opening, and a position where the first opening is closed.

In the first feature, the second cylindrical member is configured of a single layer formed with the second opening and the third opening.

In the first feature, the second cylindrical member is configured of two layers being relatively displaceable, the second opening is formed on one layer and the third opening is formed on another layer.

In the first feature, the first cylindrical member is in a fixed position, and the second cylindrical member is formed be rotatably displaceable in a circumferential direction of the second cylindrical member.

In the first feature, the first cylindrical member is in a fixed position, and the second cylindrical member is formed to be slidably displaceable in an axis direction of the second cylindrical member.

In the first feature, the heat source is formed of iron powder of 40 to 60 wt%, activated carbon of 10 to 30 wt%, water of 10 to 30 wt%, and sodium chloride of 0.5 to 7 wt%, without exceeding 100 wt% in sum.

In the first feature, the flavor generating body is a compact including tobacco.

### [BRIEF DESCRIPTION OF DRAWINGS]

[Fig. 1] Fig. 1 is a schematic view illustrating an enlarged configuration of a longitudinal cross section of a non-burning flavor inhaler according to a first embodiment of the present invention.
[Fig. 2] Fig. 2(a) is a view showing a temperature rising state, Fig. 2(b) is a view showing a temperature keeping state, and Fig. 2(c) is a view showing a suspended state where a contact of a heat source and an air is suspended, for the non-burning flavor inhaler shown in Fig. 1.
[Fig. 3] Fig. 3 is a view illustrating a non-burning flavor inhaler according to a second embodiment of the present invention.
[Fig. 4] Fig. 4 is a view illustrating a non-burning flavor inhaler according to a third embodiment of the present invention.
[Fig. 5] Fig. 5 is a view illustrating a non-burning flavor inhaler according to a fourth embodiment of the present invention.
[Fig. 6] Fig. 6 is a view showing a relationship between a heating temperature and an amount of provided menthol for samples of flavor inhalers for examination.
[Fig. 7] Fig.7 is a view showing a state of films having different ventilation rate of air.
[Fig. 8] Fig. 8 is a view for explaining a heat source manufactured for the examination.
[Fig. 9] Fig. 9 is a graph showing a summary of temperature profiles suitable for a heat source.

### [DESCRIPTION OF EMBODIMENTS]

Hereinafter, a preferable embodiment of the present invention is described with reference to the drawings.

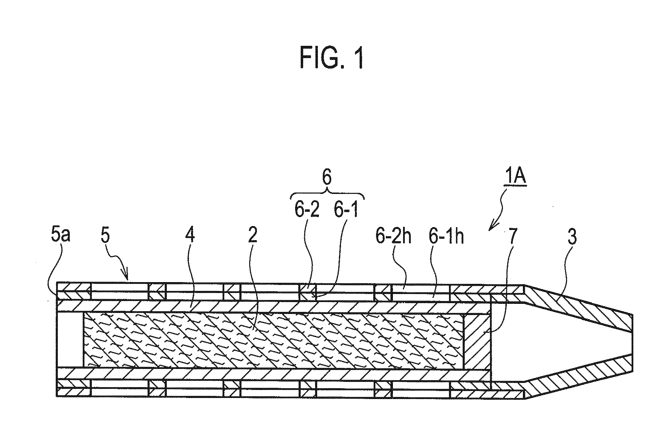

Fig. 1 is a schematic view illustrating an enlarged configuration of a longitudinal cross section of a non-burning flavor inhaler 1A according to a first embodiment of the present invention.

The non-burning flavor inhaler 1A includes a flavor generating body 2, a mouthpiece 3 for inhaling a flavor by a user, a heat source 4 raising a temperature of the flavor generating body 2, a casing member 5 holding the flavor generating body 2, the mouthpiece 3, and the heat source 4. An external form of non-burning flavor inhaler 1A is formed into a rod shape similar to a cigarette or a cigar. The casing member 5 is provided with an air adjusting structure 6.

The flavor generating body 2 is approximately a rod shape, for example, and is a member to impart a flavor to an air flow entering form an one end side and exiting from an opposite end side. The mouthpiece 3 for inhaling by the user is provided at the opposite end side of the flavor generating body 2. The heat source 4 produces heat by chemical reaction, and is arranged to cover an outer peripheral of the flavor generating body 2 to raise the temperature of the flavor generating body 2. The casing member 5 is a holder which holds the flavor generating body 2, the mouthpiece 3, and the heat source 4, and therefore the external form of the non-burning flavor inhaler 1A is kept in the rod shape.

Especially, the heat source 4 produces heat by oxidation reaction without burning, and the casing member 5 is a structure including the air adjusting structure 6 which reversibly changes an area of contact between the heat source 4 and an air used for the oxidation reaction.

Here, the term "reversibly" means that a contact state between the heat source 4 and the air can be mutually changed between a temperature rising state, a temperature keeping state, and a suspended state, as described later. That is, the term "reversibly" means that the contact area between the heat source 4 and the air can be mutually changed among at least two kinds of area sizes.

An outline shape of the casing member 5 has a cylindrical hollow shape, the casing member 5 houses the flavor generating body 2 covered by the above described heat source inside thereof. A one end thereof is an opening 5a and the mouthpiece 3 is fixed on an opposite end thereof. A filter 7 may be arranged at the mouthpiece 3 side of the flavor generating body 2 if needed. The filter may be adopted that is formed of an acetate fiber, a paper or a non-woven fabric similar to the cigarette, for example.

The casing member 5 has the air adjusting structure 6 adjusting a contact ratio (ventilation degree) between the heat source 2 and the air, before explaining it in detail, the flavor generating body 2 and the heat source 4 preferable for the flavor inhaler will be explained firstly.

The flavor generating body 2 includes a flavor generating source and it may be a granular tobacco body, for example. However, the flavor generating body 2 is not limited to this. The flavor generating source may adopt materials arbitrarily selected that generates a flavor by temperature rise and makes user relaxed. The flavor generating body 2 may be configured by filling the flavor generating source inside the heat source directly, but it may be a wrapped body where the non-woven fabric or the like having the ventilation characteristics covers an outer peripheral of the wrapped body, in view of manufacturing efficiency and handling convenience, and further in view of replacement convenience by the user on the assumption of repeating use. Further, it may be a solid body formed in to a rod shape by use of a process like extrusion of the flavor generating source itself. Such flavor generating source formed into the rod shape can improve the handling convenience since it is easy for the user to handle it.

When the granular tobacco body is used as the above flavor generating source, a grain size of a granular material is preferably 0.2mm or more and 2.0mm or less and a fill ration of the granular material is preferably 250mg or more and 1000mg or less. Further, it may include a flavor such as menthol and a flavor stabilizing agent such as propylene glycol or MCT (medium-chain triglyceride).

When the flavor generating source is formed into a compact, a shape forming may be preferably performed by blending a binder such as CMC (carboxymethylcellulose) having a content rate of 2% or more and 5% or less to the granular tobacco body, for example. The structure may be preferably a honeycomb shape in view of the ventilation characteristics and an increase of surface area.

The heat source 4 is not limited if it utilizes the oxidation reaction heat using oxygen included in the air (atmosphere) as a heating method. However, it is preferable to use the oxidation reaction heat of iron powder in view of availability of material and handling convenience.

When a tobacco material is used as the flavor generating source, a heated temperature is preferably 60 degrees C or more and 70 degrees C or less. If the heated temperature is 60 degrees C or more, it is possible to inhale the sufficient flavor equivalent to a menthol cigarette sold in a market, for example. These points will be described later raising concrete examples.

If the heated temperature is less than 60 degrees C, amount of the flavor component from the flavor generating body tends to become insufficient. On the contrary, if the heated temperature is more than 70 degrees C, amount of the flavor component rapidly increase and it is difficult to provide suitable amount of the flavor to the user. In case that the heated temperature exceeds 70 degrees C, it is not preferable because some action such as an arrangement of a heat shield material is required to shield the excessive heat when the user using it in a state of holding it in his or her hand.

The heat source 4 may be a heat generation body having compositions such as iron powder, activated carbon, water, and sodium chloride, for example. In detail, the heat source 4 is preferably formed of the iron powder of 40 to 60 wt%, the activated carbon of 10 to 30 wt%, the water of 10 to 30 wt%, and the sodium chloride of 0.5 to 7 wt%, under the condition that the sum of the compositions do not exceed 100 wt%.

For example, when using the activated carbon having a specific surface area of 1700m²/g, a ratio of the iron powder, the activated carbon, the water, and the sodium chloride is preferably 49:22:25:4 in view of a rising rate of heat and heat keeping characteristic. On the other hand, when using the activated carbon having a specific surface area of 2300m²/g, a preferable weight % of the iron powder, the activated carbon, the water, and the sodium chloride may be 54:14:27:5.

The heat source 4 may have a sheet shape which can cover (roll up) the outer peripheral of the flavor generating body having the rod shape, it may be a packed body filled with the granular material having the above compositions inside a packing material such as a pouch. Alternately, it may be a shaped body formed by flattening a mixture into the sheet shape after mixing the above compositions. When forming the heat source by such granular material, a grain size of the iron powder is preferably 30µm or more and 200µm or less and a grain size of the activated carbon may be 5µm or more and 50µm or less. The total weight of the heat source is preferably 3g or more and 8g or less.

It is preferable to have a shape that can obtain a sufficient contact area with the air in view of keeping necessary amount of oxygen provided to the heat source, and also it is preferable to have a structure and an arrangement that can effectively transfer the generated heat to the flavor generating body 2 side. Accordingly, the heat source has configuration of cylindrical hollow to cover the outer peripheral of the flavor generating body 2, and the casing member 5 arranged outside the heat source 4 has the air adjusting structure.

Next, the air adjusting structure will be explained that is provided on the casing member 5, and adjusts the contact ratio (ventilation degree) of the air as a result of changing the contact area of the air (oxygen) used for the oxidation reaction in the heat source 4.

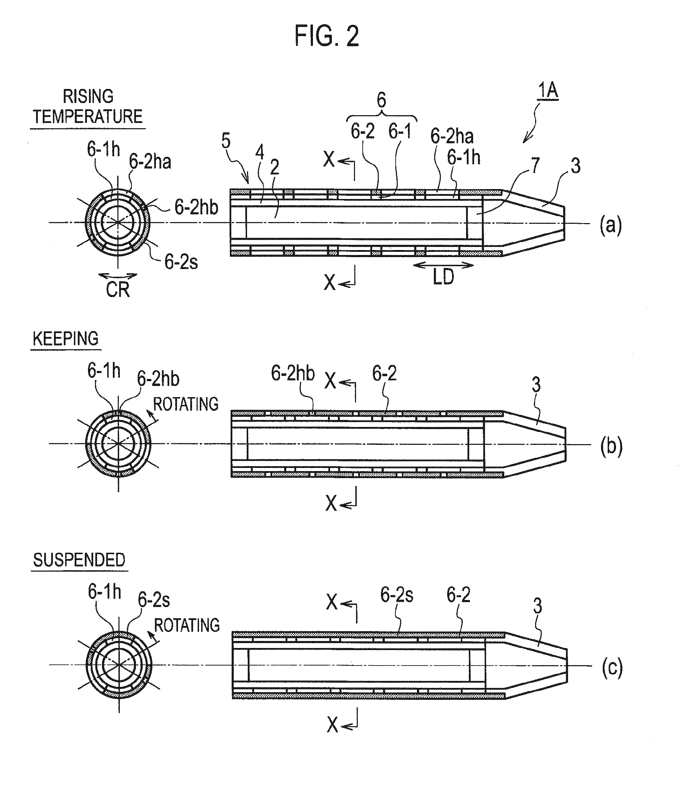

Further, Fig. 2 will be referred. It is to be noted that Fig. 1 shown previously is an enlarged view showing an arrangement of each constituent element for the sake of clarity, and corresponds to Fig. 2 (a). Also, as described later, Fig. 2 (a) is view showing a temperature rising state in which a contact between a heat source and air is increased in area; Fig. 2 (b) is a view showing temperature keeping state; and Fig. 2 (c) is a view showing a suspended state in which the contact between the heat source and air is suspended, respectively. In addition, at the left side of the respective one of Fig. 2 (a) to Fig. 2 (c), a cross section taken along the line X-X is shown.

Although the casing member 5 functions as a holding structure to hold in contact with the outer circumference of the heat source 4, this casing member also functions as the air adjusting structure. This casing member is provided with: an inner cylinder layer 6-1 as a first cylindrical member; and an outer cylinder layer 6-2 as a second cylindrical member which is provided to cover the inner cylinder layer 6-1, and is formed to be relatively displaceable, preferably slide with respect to the inner cylinder layer. The non-burning type flavor inhaler 1A shown in Fig. 1 is arranged so that the outer cylinder layer 6-2 is rotatable on the inner cylinder layer 6-1 in a circumferential direction CR.

In the inner cylinder layer 6-1, the mouthpiece 3 is also fixed together with the heat source 4. In the inner cylinder layer 6-1, an opening 6-1h for supplying air to the heat source 4 existing therein is provided. In the example shown herein, there is illustrated a case in which a total of eight openings 6-1h are arranged in four lines along an axial direction LD and are arranged in two lines so as to be opposed to each other in a radial direction perpendicular to the axial direction LD (with an angle of 180 degrees in the circumferential direction CR with respect to an axial center).

In addition, on the outer cylinder layer 6-2 provided on the inner cylinder layer 6-1, an opening 6-2h is provided in correspondence with the opening 6-1h. The inner cylinder layer 6-1 mentioned previously is a structure as a fixed heating body covering layer provided to cover the heat source 4, and the outer cylinder layer 6-2 can be regarded as a rotating cover to make displacement (slide) thereon in a circumferential direction.

In the opening 6-1h provided in the inner cylinder layer 6-1, an opening area as a maximum area to bring the heat source 4 into contact with air is provided. More specifically, it is desirable that the opening area be defined under a condition that the heating temperature described previously at the time of use, for example, is speedily obtained in order of 60 to 70 degrees C.

There are two kinds of openings 6-2h provided in the outer cylinder layer 6-2 with respect to the foregoing description. Although this matter was not clear in Fig. 1, it can be verified by way of Fig. 2. That is, in the openings 6-2h, an opening 6-2ha having an opening area which is approximately similar to that of the opening 6-1h; and an opening 6-2hb of an opening area which is smaller than that of the opening 6-1h are provided.

In addition, in the outer cylinder layer 6-2, there is provided a closed region 6-2s with no opening, which is capable of closing the opening 6-1h of the inner cylinder layer 6-1 so as to preclude the heat source 4 from coming come into contact with air.

According to the non-burning type flavor inhaler 1A provided with the constituent elements mentioned above, the state shown in Fig. 2 (a) in which positions of the opening 6-1h of the inner cylinder layer 6-1 and the opening 6-2ha of the outer cylinder layer 6-2 are coincident with each other with an angle of rotating the outer cylinder layer 6-2 is obtained as a temperature rising mode. In addition, the state shown in Fig. 2 (b) in which positions between the opening 6-1h of the inner cylinder layer 6-1 and the opening 6-2hb of the outer cylinder layer 6-2 are coincident with each other is obtained as a temperature keeping mode. Further, the state shown in Fig. 2 (c) in which ventilation of the opening 6-1h of the inner cylinder layer 6-1 is shut out by the closed region 6-2s of the outer cylinder layer 6-2 can be defined as a suspended mode.

The temperature rising mode mentioned above is a mode employed at the time of start of use of the non-burning type flavor inhaler 1A, and the temperature rise of the flavor generating body 2 is achieved by the rapid heat generation of the heat source. The temperature keeping mode mentioned above is a mode for optimally keeping the heat generation of the heat source while the user enjoys the flavor.

Also, the suspended mode is a mode to suspend air supply to a heat source and suspend heating of a heat source, and an oxidation reaction can be suspended partway by suspending the air (oxygen) supply. Afterwards, the non-burning type flavor inhaler 1A can be used by bringing a heat source into contact with air again. Therefore, in so far as this non-burning type flavor inhaler 1A is concerned, a user can also enjoy the flavor for multiple times and then economical use can be achieved.

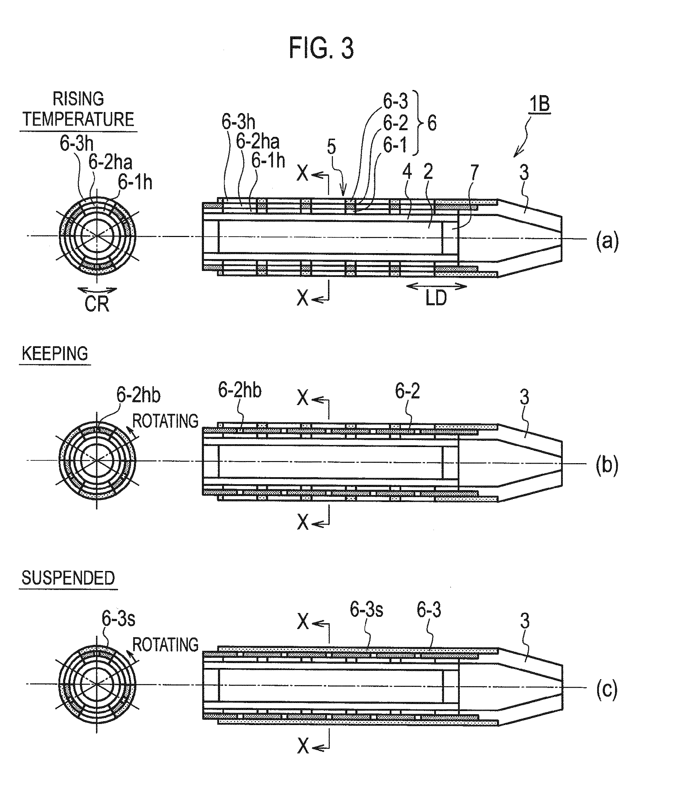

In addition, Fig. 3 is a view schematically showing a non-burning type flavor inhaler 1B according to a second embodiment of the present invention, associated with the non-burning type flavor inhaler 1A shown in Figs. 1 and 2. Fig. 3 shows a longitudinal cross-sectional configuration as to the non-burning type flavor inhaler 1B, wherein Fig. 3 (a) shows a temperature rising state, Fig. 3 (b) shows a temperature keeping state, and Fig. 3 (c) shows a suspended state, in a similar manner as that in Fig. 2.

It is to be noted that as to this non-burning type flavor inhaler 1B, a duplicate description is omitted by assigning the same reference numerals to the same constituent elements in the non-burning type flavor inhaler 1A, and differences therebetween will be mainly described.

In the non-burning type flavor inhaler 1B of Fig. 3, outside of the inner cylinder layer 6-1, outer cylinder layers are formed in two layers. That is, outside of the inner cylinder layer 6-1, a first outer cylinder layer 6-2, and further, a second outer cylinder layer 6-3 are provided.

Also, in the first outer cylinder layer 6-2, a large opening 6-2ha corresponding to the opening 6-1h and an opening 6-2hb which is smaller than the opening 6-1h are provided. In addition, in the second outer cylinder layer 6-3, a large opening 6-3h corresponding to the opening 6-1h and the opening 6-2ha is provided, and a closed region 6-3s is provided.

In addition, the first outer cylinder layer 6-2 and the second outer cylinder layer 6-3 mentioned above are formed so as to be respectively rotatable independently on the inner cylinder layer 6-1. It is to be noted that, as illustrated, as long as a left end of the second outer cylinder layer 6-3 is formed to be shorter than a left end of the first outer cylinder layer 6-2, for example, the outer cylinder layer 6-2 and the second outer cylinder layer 6-3 can be individually rotated.

In the non-burning type flavor inhaler 1B mentioned above, when all of the three layers are caused to be coincident with a large opening in Fig. 3 (a), a temperature rising mode can be obtained. In addition, when a small opening 6-2hb forms the overlapped opening along the way of rotating the first outer cylinder layer 6-2 in Fig. 3 (b), a temperature keeping mode can be obtained. Further, when ventilation is shut out by the closed region 6-3s of the rotated second outer cylinder layer 6-3 in Fig. 3 (c), a suspended mode can be obtained. Thus, in the non-burning type flavor inhaler 1B also, the temperature rising mode, the temperature keeping mode, and the suspended mode, all of which are similar to those of the non-burning type flavor inhaler 1A, can be formed.

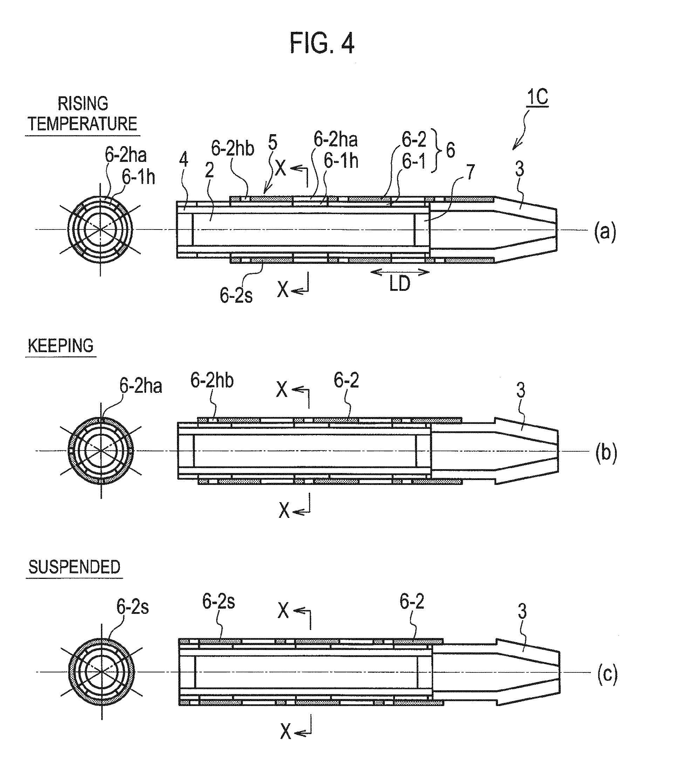

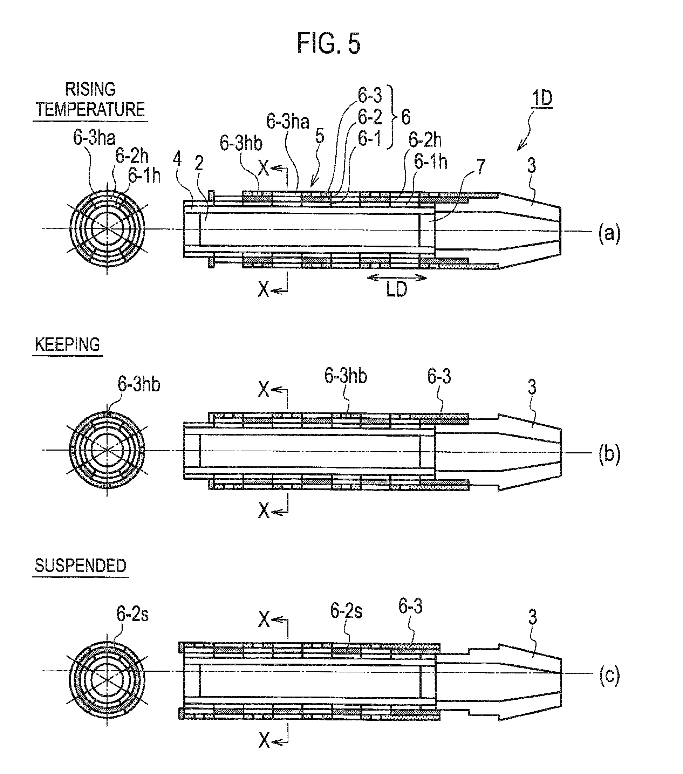

Further, Fig. 4 is a view showing a non-burning type flavor inhaler 1C according to a third embodiment of the present invention, and Fig. 5 is a non-burning type flavor inhaler 1D according to a fourth embodiment similarly.

The flavor inhaler 1C shown in Fig. 4, as in the flavor inhaler 1A described previously, is similar thereto in that an inner cylinder layer 6-1 and an outer cylinder layer 6-2 that can be relatively displaceable with respect to this inner cylinder layer 6-1 are provided. However, there is a difference therebetween in that this outer cylinder layer 6-2 is formed so as to slide on the inner cylinder layer 6-1 in an axial direction LD.

Therefore, in this outer cylinder layer 6-2, along the axial direction LD, there are provided: a large opening 6-2ha of an opening area which is approximately the same as that of the opening 6-1h on the inner cylinder layer 6-1 side; an opening 6-2hb which is smaller than the large opening 6-2ha; and a closed region 6-2s.

In the non-burning type flavor inhaler 1C mentioned above, the outer cylinder layers 6-2 are sequentially slid in the axial direction LD, whereby the state of Fig. 4 (a) in which positions of the opening 6-1h of the inner cylinder layer 6-1 and the opening 6-2ha of the outer cylinder layer 6-2 are coincident with each other is obtained as a temperature rising mode. In addition, the state of Fig. 4 (b) in which the opening 6-1h of the inner cylinder layer 6-1 is caused to slide to the opening 6-2hb of the outer cylinder layer 6-2 is obtained as a temperature keeping mode. Further, the state of Fig. 2 (c) in which ventilation of the opening 6-1h of the inner cylinder layer 6-1 is shut out by the closed region 6-2s of the slid outer cylinder layer 6-2 is obtained as a suspended mode.

Thus, in the non-burning type flavor inhaler 1C in which an outer cylinder layer is replaced with that of slide-type also, as in the non-burning type flavor inhaler 1A, a temperature rising mode, a temperature keeping mode, and a suspended mode can be formed.

Further, although the non-burning type flavor inhaler 1D according to the fourth embodiment of the present invention shown in Fig. 5, as in the flavor inhaler 1B described previously, is similar thereto in that an inner cylinder layer 6-1 and outer cylinder layers 6-2, 6-3 of two layers are provided thereon, these outer layers are slid in the axial direction LD in the same manner as that in the non-burning type flavor inhaler 1C according to the third embodiment.

Here, at the side of the first outer cylinder layer 6-2 which is the inside, an opening 6-2h corresponding to the opening 6-1h of the inner cylinder layer 6-1 and a closed region 6-2s are provided. In addition, at the side of the second outer cylinder layer 6-3 which is the outside, a large opening 6-2ha and a small opening 6-2hb are provided in correspondence with the opening 6-1h of the inner cylinder layer 6-1. In this non-burning type flavor inhaler 1D also, as in the non-burning type flavor inhaler 1C, a temperature rising mode, a temperature keeping mode, and a suspended mode can be formed.

It is to be noted that, in the slide-type noncombustible favor inhalers 1C, 1D shown in Figs. 4 and 5, a stopper to restrain a displacement may be appropriately provided at an end part (a left end) of a push, or alternatively, a structure in which when the mouthpiece 3 is rotated, an outer cylinder layer slides in an axial direction in response to this rotation may be added.

The structure to open and close an opening in the inner cylinder layer and the outer cylinder layer provided in the respective one of the non-burning type flavor inhalers 1A to 1D described hereinbefore is a preferred example of the configuration as an air adjusting structure, and as long as the structure is a structure in which a state of supplying air to the heat source 4 can be reversibly adjusted, an advantageous effect similar thereto can be attained, the structure is not limitative to those shown in Figs. 2 to 5.

It is to be noted that a material constituting the inner cylinder layer and the outer cylinder layer may be a gas impermeable film member, or alternatively, a gas impermeable resin material or the like can be preferably employed.

Also, an indicator to visualize a heating temperature of a heat source or the like can be added so as to keep track of timing for a user to adjust ventilation. In this regard, a temperature indicating material to reversibly change a color at a predetermined temperature can be preferably employed, and for example, a temperature indicating material shown in Japanese Patent No. 4175519 can be used. More specifically, there may be employed a method or the like of containing or surface-coating a temperature indicating material in part or all of an outermost provided material, or alternatively, attaching a seal including a temperature indicating material. A temperature at which a temperature indicating material generates a change in color may be set at a specific temperature in the range of 60 to 70 degrees C, or alternatively, temperature indicating materials with color change temperatures which are different from each other are arranged at a plurality of sites, whereby a temperature change in a heat source may be displayable in a stepwise manner.

Although the non-burning type flavor inhaler according to the present invention can be understood from the foregoing description, a more specific description will be given below with respect to a relationship between the flavor generating body and a heating temperature in a case where a flavor generating body including tobacco is employed and a heat source or the like which is preferable thereto (a relationship between the flavor generating body and the heating temperature).

As described below, a flavor generating body was fabricated presuming a substitute of menthol cigarette. A flavor source was obtained by mixing 320 mg of tobacco powder, 60 mg of menthol, and 12 mg of MCT (Medium Chain Triglycerides) and peppermint flavor, and a flavor source was obtained by filling the mixture in PTA (tetra fluoroethylene / perfluoroalkyl vinyl ether copolymer) tuber with an internal diameter of 8 mm and a length of 45 mm. This flavor source was contained and sealed in bag made of EVOH (ethylene / vinyl alcohol copolymer resin), the sealed flavor source was stored for 3 days under a condition of 22 degrees C in temperature and 60% in humidity, and the stored flavor source was obtained as a test sample for confirming an effect of a flavor generating body.

Sampling and quantitative analysis were carried out, focusing on a flavor components (menthol) of the flavor generating body mentioned above. As to the sample mentioned above, sampling of the respective flavor component was carried out at a respective one of heating temperatures of 50 degrees C, 60 degrees C, 70 degrees C, and 80 degrees C. Here, on the presumption of applying to a structure in the non-burning type flavor inhaler mentioned previously, the flavor generating body is set so as to cover an outer circumferential part by a heater heated at a predetermined temperature, the set flavor generating body was kept for 5 minutes, a material temperature reached equilibrium, and thereafter, sampling was carried out. Inhalation was carried out by one set of smoking devices (inhalation capacity: 55 ml, inhalation time: 2 seconds, inhalation intervals: 30 seconds), and the flavor component s for 10 puffs were collected by an impinger which is a liquid collecting device (extracted liquid: 10 ml of ethanol). The collected liquid was quantitatively measured by employing a GC/MS (gas chromatography mass analyzer), and a menthol supply quantity in one puff was computed.

At this juncture, as Comparative Examples, with respect to 6 kinds of commercially available menthol cigarette, sampling was carried out under a condition similar to the above mentioned condition, and quantitative measurement was carried out by employing the GC/MS, and a menthol supply quantity in one puff was carried out. In these commercially available menthol cigarettes, an approximate menthol supply quantity in one puff was about 100 to 250µg /puff.

With respect to the commercially available menthol cigarette mentioned above, Table 1 below shows a menthol supply quantity in one puff of the sample mentioned above at a respective one of the heating temperatures of 50 degrees C, 60 degrees C, 70 degrees C, and 80 degrees C.

**[Table 1]**

| Heating temperature | Menthol supply quantity (µg/puff) |
|---|---|
| 50 degrees C | 90 |
| 60 degrees C | 189 |
| 70 degrees C | 276 |
| 80 degrees C | 635 |

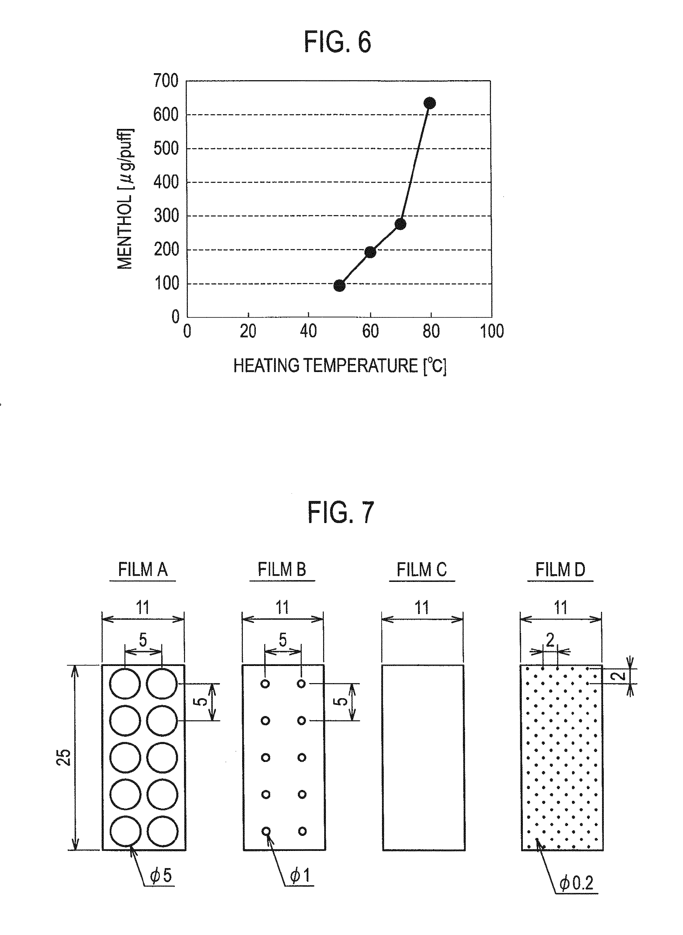

In addition, Fig. 6 is a view showing a relationship between heating temperatures and menthol supply quantities as to test samples of the flavor generating body.

In accordance with the Table 1 and Fig. 6 shown above, it is possible to confirm that menthol supply similar to that of the commercially available menthol cigarette can be achieved at a heating temperature of order of 60 degrees C. In addition, if the heating temperature exceeds 70 degrees C, a rapid increase of menthol supply quantity is observed, and in particular, the menthol supply quantity at 80 degrees C is very high, which is about 3 to 5 times of that of the commercially available menthol cigarette. Therefore, it is desirable that the heating temperature of the non-burning type flavor inhaler presuming a substitute of menthol cigarette be 70 degrees C or less.

Next, as described above, it is sufficient if heating of a heat source be controlled so as to meet conditions that: 1) the flavor generating body that is preferable to be heated at 60 to 70 degrees C be speedily heated at the time of start of use; 2) the flavor generating body that is capable of being maintained at the suitable temperature for heating the flavor generation over use; and temperature profiles of 1) and 2) can be reproduced at multiple times. This matter will be described below. A heat source pouch (a heat source and a temperature profile) was fabricated as follows.

A heat generation body was obtained by mixing 400 mg of iron powder (45µm 99.9% of iron powder manufactured by Wako Pure Chemical Industries, Ltd.) and 100 mg of active carbon (Kuraray Coal SA2300 average particle size 6µm available from Kuraray Chemical Co, Ltd.).

This heat generation body was contained in pouch of 11 mm in width and 25 mm in length fabricated by attaching a polypropylene film (Mini-grips 0.04 mm in thickness manufactured by Seisan Nipponsha) and a hydrophilic polypropylene non-woven cloth (PC0070-OEM manufactured by Kuraray Kuraflex Co., Ltd.).

In addition, on the hydrophilic polypropylene non-woven cloth side mentioned above, 15% of sodium chloride water solution (sodium chloride, reagent, special class, manufactured by Wako Pure Chemical Industries, Ltd.) was added by 212µl, the thus added solution was stored in one night (for 12 hours) under a sealing condition in which transfer of oxygen was shut out, and then, a heat source pouch was obtained.

Next, a member for adjusting a contact between the heat source and air was fabricated. It is to be noted that this member presumes an air adjusting structure in the non-burning type flavor inhaler of the present invention described above. A heated state of the heat source in change of the contact between the heat source and air is verified, and a preferred temperature profile is specified.

As shown in Table 2 below, 4-level films A to D which are different from each other in ventilation characteristics with respect to air were prepared.

**[Table 2]**

| | Material | Opening diameter (mm) | Opening intervals (mm) | Number of openings (quantity) | Film area (mm²) | Opening percentage (%) |
|---|---|---|---|---|---|---|
| Film A | Polypropylene | 4.0 | 5 | 10 | 275 | 46 |
| Film B | Polypropylene | 1.0 | 5 | 10 | 275 | 3 |
| Film C | Cellophane tape | 0.0 | - | 0 | 275 | 0 |
| Film D | Polypropylene | 0.2 | 2 | 138 | 275 | 2 |

It is to be noted that in table 2 given above, the opening percentage (%) was computed by opening percentage (%) = (Total opening area (mm²) / film area (mm²) x 100.

As the films A and B mentioned above, gas impermeable polypropylene films (Mini Grips 0.04 mm in thickness manufactured by Seisan Nipponsha) were used, openings of which were provided by a belt punch (TPS-8S manufactured by Trusco Nakayama Corporation and SH11 half-circle drive punch 1 mm manufactured by Fujiwara Sangyo Co., Ltd.)

As the film C, a cellophane tape (scotch transparent adhesive tape, trade name: TomeiBishoku CC1220-BXJ manufactured by Sumitomo 3M Limited) was used.

As the film D, a polypropylene film (FOH20/P02 manufactured by OhE Chemicals Inc.) was used.

A planer appearance of a respective one of the films A to D in an enlarged view was shown in Fig. 7.

The verification test as described below was carried out by employing the heat source (hear source pouch) fabricated as described above.

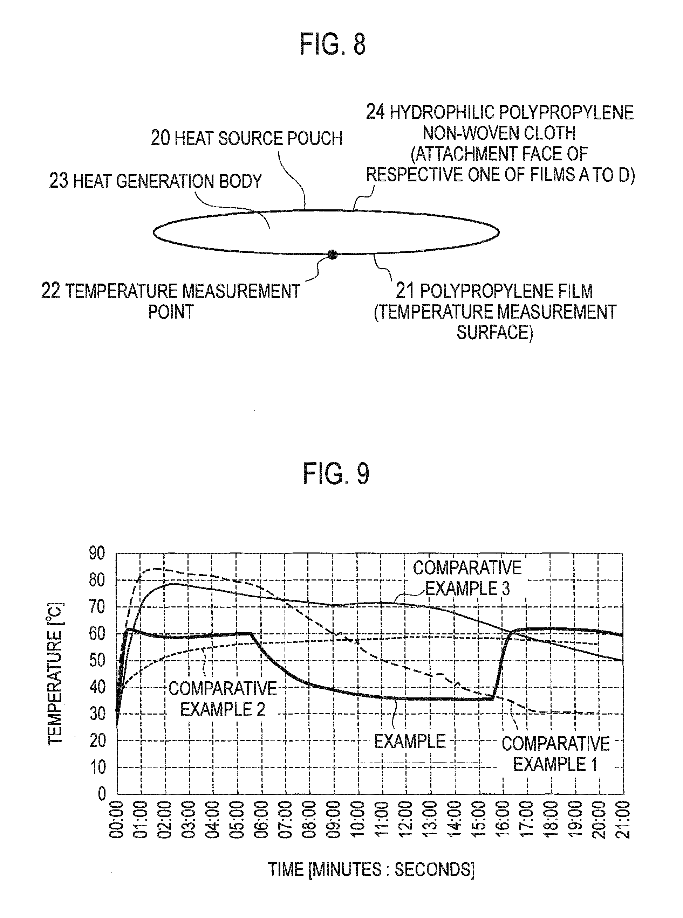

Temperature measurement, as shown in Fig. 8, was carried out by attaching a K-type thermocouple (SF30Kϕ0.25 x 100-U-2-COS(P) manufactured by Fukuin Tokushu Kinzoku, Inc.) while a center part at the side of the gas impermeable polypropylene film 21 of a heat source pouch 20 is defined as a temperature measurement point 22.

In the heat source pouch 20, an opposite side to the heat generation body 23 as a heat source and the polypropylene film 21 is employed as a hydrophilic polypropylene non-woven cloth 24, and this side is an attachment face of a respective one of the films A to D.

After temperature measurement had been started (after a heating reaction had started), in accordance with a temperature of the heat source pouch and an elapse of time, an operation of changing kinds of films to be attached was carried out as follows.

1) From the start of measurement up to a time point at which a heating temperature of 60 degrees C had been reached, only the film A with the largest opening percentage was attached to the face of the hydrophilic polypropylene non-woven cloth 24 with respect to the heat source pouch 20.
2) For 5 minutes after the time point at which the heating temperature of 60 degrees C had been reached, the film B with a small opening percentage was attached onto a top face of the film A.
3) For 10 minutes after the completion of step 2), the film C was attached onto a top face of the film B.
4) From the time of completion of step 3) up to a time point at which a heating temperature of 60 degrees C had been reached again, the film and the film C were removed, and only the film A was attached.
5) For 5 minutes after a time point at which the temperature raised up to 60 degrees C again, a film B having a small opening percentage was attached onto the top face of the film A.

In response to the verification test mentioned above, the comparative tests mentioned below were also carried out.

As Comparative Example 1, measurement was carried out as to a case in which only the film A was attached onto a surface of a hydrophilic polypropylene non-woven cloth as a heat source pouch all over the measurement temperatures. That is, the sampling conditions were similar to those of the steps 1) to 4) above.

As Comparative Example 2, measurement was carried out as to a case in which only the film A was attached onto the surface of the hydrophilic polypropylene non-woven cloth of the heat source pouch and then the film B was attached onto the top face thereof all over the temperature temperatures. That is, the sampling conditions were similar to the steps 2) to 5) mentioned above.

As Comparative Example 3, measurement was carried out as to a case in which only the film D was attached onto the surface of the hydrophilic polypropylene non-woven cloth all over the measurement temperatures.

Fig. 9 is a graph collectively showing a verification test example (a preferred temperature control example) in a case where operation has been made as described above and a graph depicting temperature profiles of Comparative Examples 1 to 3 shown above in all.

In Comparative Example 1 above, a temperature reaches 60 degrees C which is the suitable temperature for flavor inhaler within about 30 seconds after start of reaction, and an appropriate temperature rising rate was indicated. However, a heat source temperature exceeded 80 degrees C, and a strong flavor would be supplied within a short period of time. Therefore, this is unsuitable as a heat source of the flavor inhaler.

In addition, in Comparative Example 2, although the achieved temperature reaches 60 degrees C which is the suitable temperature for the flavor inhaler, the temperature rising rate was very low. Time intervals of the order of 10 minutes were required for raising the temperature up to 60 degrees C, and thus, this is unsuitable as the heat source of the flavor inhaler.

Further, in Comparative Example 3 also, the relevant temperature was approximate to that in Comparative Example 1, this is unsuitable as the heat source of the flavor inhaler.

In connection with the foregoing description, in the case of the verification test example mentioned above, the following temperature profiles were indicated.

1) About the temperature reached 60 degrees C within about 30 seconds after measurement had been started.
2) Afterwards, the temperature was kept about 60 degrees C for 5 minutes.
3) Subsequent to the step 2) mentioned previously, when the time period of 10 minutes had been taken as a time to suspend air supply, heating suspended and then the heat source temperature lowered to a normal temperature.
4) Subsequent to the step 3) mentioned previously, when the heating body had been brought into contact with air, the temperature reached 60 degrees C within about 40 seconds.
5) Afterwards, the temperature was kept at 60 degrees C for 5 minutes.

In according with the steps 1) and 2) mentioned above, at a time point of the start of use, ventilation for a heat generation body is greatly ensured and then the ventilation is limited at a stage at which a temperature reached 60 degrees C, whereby heating is carried out to speedily temperature rise immediately after the start of use, and however, an excessively high temperature is not reached, and it is possible to confirm that a temperature can be kept at 60 degrees C, which is the suitable temperature for the flavor inhaler, for at least for 5 minutes.

In addition, it was confirmed that it can be used for multiple times by suspending the heat generation of the heat generation body when not in use and contacting the air to the heat generation body again, in accordance with the steps 3) to 5) mentioned above.

In view of the foregoing description, preferred modes to reversibly control air so as to be brought into contact with the heating body are:
a) To set a condition (a degree of ventilation) of a contact area of an air to the heat generation body so that an equilibrium temperature which is slightly higher (by the order of 2 or 3 degrees C) than a target temperature (hereinafter, 60 degrees C) at the time of start of use;
b) To reduce the degree of ventilation to a predetermined quantity after the target temperature has been reached and then keep the target temperature; and
c) To completely shut out air coming into contact with the heat generation body at the time of completion of use.

As in the modes a) to c) above, it was verified that the conventional problem pointed out previously is solved by appropriately changing the state of ventilation for the heat generation body, heating is carried out to speedily rise up to a temperature suitable for generation of flavor at the time of start of use and then the relevant temperature is kept at a temperature suitable for supply thereof, and it is possible to achieve a temperature profile suitable for the flavor inhaler for which use of a plurality of times is possible. Although in the foregoing description, there was experimentally shown a case of controlling the air (oxygen) with which a heat source is brought into contact by way of the films A to C, in so far as the non-burning type flavor inhaler according to the present invention described previously is concerned, it is possible to achieve a preferred temperature profile in the same manner as that verified by the verification test, by way of the air adjusting structure described previously.

The present invention described hereinbefore is not limitative to the embodiments described previously. The present invention can be variously modified and carried out without departing from the spirit of the invention.

In the non-burning type flavor inhaler according to the present invention, a flavor generating body and a heat source are made exchangeable, and a casing member and a mouthpiece as other constituent elements can be used repeatedly, whereby economical use is possible.

The present non-burning type flavor inhaler may be achieved in size analogous to a cigar (for example, 70 mm to 100 mm in length, 12 mm to 16 mm in outer diameter) from the viewpoint of sustaining sufficient flavor supply and heating.

Also, an opening provided in an inner cylinder layer or an outer cylinder layer to achieve the air adjusting structure mentioned previously may be any shape such as a circular shape or a rectangular shape, or alternatively, an elliptical shape without being limitative thereto, as long as it is possible to adjust the air coming into contact with a heat source. In addition, although in the foregoing description, there was illustrated a case in which a plurality of openings are provided in each cylinder layer, one opening may be provided.

In detail, an overlapping area (that is, a contact area between a heat source 4 and air) in which an opening (hereinafter, a first opening) provided in a first cylindrical member (for example, a first inner cylinder layer 6-1) and an opening (hereinafter, a second opening) provided in a second cylindrical member (for example, an outer cylinder layer 6-2) overlap with each other may be capable of being changed among at least two or more kinds of area sizes. In other words, in a case where a plurality of positions are present as relative positions between the first cylindrical member and the second cylindrical member, the overlapping area in which the openings of the respective cylindrical members overlap with each other at the respective positions (that is, the contact area between a heat source 4 and air) may be different from each other. Therefore, the shapes of the first opening and the second opening do not need to be circular shapes, and may be slit shapes or may be elliptical shapes. It is sufficient if the first cylindrical member and the second cylindrical member have a configuration being relatively displaceable so as to change the overlapping area of the first opening and the second opening among at least two or more kinds of area sizes.

It is to be noted that at least the two or more kinds of area sizes may include a zero area. Also, as described in the embodiments, it is preferable that the first cylindrical member and the second cylindrical member have a configuration being relatively displaceable so as to change the overlapping area of the first opening and the second opening among three kinds of area sizes.

Further, the second cylindrical member has openings having different areas from each other (for example, the opening 6-2ha and the opening 6-2hb). However, the embodiments are not limitative thereto. That is, the overlapping area in which the first opening and the second opening overlap with each other may be capable of being changed, and thus, the first cylindrical member may also have only an opening having one kind of area.

Furthermore, the non-burning type flavor inhalers 1C, 1D shown in Figs. 4 and 5 are intended to slide an outer cylinder layer along an axial direction, and thus, the shapes of transverse cross sections do not need to be circular shapes, and may be any shapes such as polygonal shapes or flat plate shapes.

In detail, the shape of the flavor generating body 2 is not limitative to a rod-like shape (a cylindrical pillar shape), and may be a polygonal pillar shape or a flat plate shape. It is sufficient if the heat source 4 be provided at a position at which the flavor generating body 2 can be heated, and thus, the shape of the heat source 4 may also be any shape.

For example, the heat source 4 may be laminated to be adjacent to the flavor generating body 2. It is sufficient that the first cylindrical member and the second cylindrical member house the flavor generating body 2 and the heat source 4, and thus, the shapes of the first cylindrical member and the second cylindrical member may also be hollowed shapes having polygonal cross sections without being limitative to the cylindrical shapes.

Still furthermore, as described above, it is sufficient if the first cylindrical member and the second cylindrical member have a configuration being relatively displaceable so as to change the overlapping area of the first opening and the second opening. Therefore, in a case where the non-burning type flavor inhaler has a rectangular parallelepiped shape, the first cylindrical member may have a box-like shape, and the second cylindrical member may have a hollowed shape having a rectangular cross section.

The entire contents of Japanese Patent Application No. 2012-012003 (filed in January 24, 2012) are incorporated in the specification of the present application by reference.

### INDUSTRIAL APPLICABILITY

According to the non-burning type flavor inhaler of the present invention, the air adjusting structure is included while the oxidation reaction heat is employed as the heat source, and thus, it is possible to achieve the preferable temperature profile such that the temperature is rapidly rise up to the suitable temperature for generating the flavor component, and thereafter, the temperature is kept at the suitable temperature for supplying the flavor component by controlling the contact ratio between the heat source and the air. Therefore, it is possible to provide the non-burning type flavor inhaler which is capable of reliably providing the flavor in a state where the heat source is kept at the suitable temperature while the rapid temperature raise of the heat source can be achieved at the time of use by the user, and further, which is can be used for multiple times.

## Claims

1. A non-burning type flavor inhaler comprising:
a flavor generating body imparting a flavor to an air flow entering form an one end side and exiting from an opposite end side,
a mouthpiece for inhaling by a user arranged at the opposite end side,
a heat source producing heat by oxidation reaction and raising a temperature of the flavor generating body, and
a casing member holding the flavor generating body, the mouthpiece, and the heat source, wherein
the casing member includes an air adjusting structure which reversibly changes an area of contact between the heat source and an air used for the oxidation reaction.

2. The non-burning type flavor inhaler according to claim 1, wherein
the air adjusting structure includes;
a first cylindrical member housing the heat source and the flavor generating body, the first cylindrical member having a first opening, and
a second cylindrical member provided at an outside of the first cylindrical member, the second cylindrical member having a second opening, and
the first cylindrical member and the second cylindrical member have a configuration being relatively displaceable so as to change an overlapping area of the first opening and the second opening among at least two kinds of area sizes.

3. The non-burning type flavor inhaler according to claim 1, wherein
the heat source is arranged to cover an outer peripheral of the flavor generating body, and
the casing member is arranged to cover the heat source.

4. The non-burning type flavor inhaler according to any one of claims 2 and 3, wherein
the air adjusting structure includes;
a first cylindrical member provided to cover the heat source, the first cylindrical member having a first opening for contacting the air to the heat source,
a second cylindrical member having a second opening having an opening area equivalent to the first opening and a third opening having an opening area smaller than the first opening, and
the first cylindrical member and the second cylindrical member have a configuration being relatively displaceable to a position where the second opening overlaps the first opening, a position where the third opening overlaps the first opening, and a position where the first opening is closed.

5. The non-burning type flavor inhaler according to claim 4, wherein
the second cylindrical member is configured of a single layer formed with the second opening and the third opening.

6. The non-burning type flavor inhaler according to claim 4, wherein
the second cylindrical member is configured of two layers being relatively displaceable, the second opening is formed on one layer and the third opening is formed on another layer.

7. The non-burning type flavor inhaler according to any one of claims 4 to 6, wherein
the first cylindrical member is in a fixed position, and
the second cylindrical member is formed to be rotatably displaceable in a circumferential direction of the second cylindrical member.

8. The non-burning type flavor inhaler according to any one of claims 4 to 6, wherein
the first cylindrical member is in a fixed position, and
the second cylindrical member is formed to be slidably displaceable in an axis direction of the second cylindrical member.

9. The non-burning type flavor inhaler according to any one of claims 1 to 8, wherein
the heat source is formed of iron powder of 40 to 60 wt%, activated carbon of 10 to 30 wt%, water of 10 to 30 wt%, and sodium chloride of 0.5 to 7 wt%, without exceeding 100 wt% in sum.

10. The non-burning type flavor inhaler according to any one of claims 1 to 9, wherein
the flavor generating body is a compact including tobacco.
